# EUROPEAN PATENT SPECIFICATION

(11) **EP 2 401 979 B1**
(45) Date of publication and mention of the grant of the patent: **20.04.2016**
(21) Application number: 11177389.1
(22) Date of filing: 16.07.2008
(51) Int. Cl.: A61B 18/14, A61B 34/10, A61B 90/00, A61B 18/18, A61B 17/34, A61B 18/02

(54) **Systems and methods for thermally profiling radiofrequency electrodes**
Systeme und Verfahren zur Wärmeformgebung von Hochfrequenz-Elektroden
Systèmes et procédés de profilage thermique d'électrodes à radiofréquence

(30) Priority: 16.07.2007 US 879061
(43) Date of publication of application: 04.01.2012
(62) Divisional of application: 08012829.1
(73) Proprietor: Covidien AG, 8212 Neuhausen am Rheinfall (CH)
(72) Inventor: Podhajsky, Ronald J., Boulder, CO Colorado 80301 (US)
(74) Representative: Soames, Candida Jane

(56) References cited:
- WO-A2-2006/042117

## Description

### BACKGROUND

### 1. Technical Field

The present disclosure relates to systems and methods for providing radiofrequency ("RF") energy to biological tissue and, more particularly to systems and methods for thermally profiling radiofrequency electrodes used in surgical procedures using RF energy.

### 2. Background of Related Art

The use of radiofrequency energy ("RF energy") and, in particular, radiofrequency electrodes ("RF electrodes") for ablation of tissue in the body or for the treatment of pain is known. Generally, such RF electrodes (e.g., probes, resistive heating elements and the like) include an elongated cylindrical configuration for insertion into the body to target tissue which is to be treated or ablated. The RF electrodes can further include an exposed conductive tip portion and an insulated portion. Accordingly, when the RF electrode is connected to an external source of radiofrequency power (e.g., an electrosurgical generator), heating of tissue occurs near and around the exposed conductive tip portion thereof, whereby therapeutic changes in the target tissue, near the conductive tip, are created by the elevation of temperature of the tissue.

The use of thermal therapy in and around the spinal column is also known. It is desirable to treat the posterior or posterior/lateral portion of the intervertebral disc for the indication of mechanical degeneration of the disc and discogenic back pain. Pain can be derived from degeneration or compression of the intervertebral disc in its posterior or posterior/lateral portions. There is some innervation of the intervertebral disc near the surface of the disc and also within its outer portion known as the annulus fibrosis. Mechanical damage such as fissures or cracks within the disc caused by age or mechanical trauma may result in disc innervation which is believed to be associated with painful symptoms.

Heating in an intervertebral disc to relieve such painful symptoms is described in U.S. Pat. No. 5,433,739 and U.S. Pat. No. 5,571,147, both to Sluijter et al., the entire contents of each of which are incorporated herein by reference. In these patents, electrodes are described in either radiofrequency or resistive thermal heating of all or a portion of the intervertebral disc. Straight, curved, and flexible-tipped electrodes are described for this purpose.

In U.S. Pat. No. 6,007,570 to Sharkey there is disclosed an intervertebral disc apparatus for the treatment of an intervertebral disc. The apparatus includes a catheter having an intradiscal section in the form of a conventional helical coil. In use, the intradiscal section is advanced through the nucleus pulposus and is manipulated to navigate within the nucleus along the inner wall of the annulus fibrosis. An energy delivering member incorporated into the apparatus adjacent the intradiscal section supplies energy to treat the disc area.

WO-A-2006/042 117 discloses a system according to the pre-amble of claim 1.

A continuing need exists for improved electrosurgical and particularly RF energy procedures which utilize thermal profiling of radiofrequency electrodes for placement of the radiofrequency electrode and the visualization of the area and/or zone of treatment of the radiofrequency electrode. A continuing need also exists for improved systems for thermally profiling radiofrequency electrodes used in surgical procedures using RF energy.

### SUMMARY

The present invention is provides a system according to claim 1. Preferred embodiments are defined in the dependent claims.

A system for thermal or electromagnetic treatment of a target surgical site, according to one particular embodiment of the present disclosure, includes a cannula having proximal and distal ends and a probe for energy delivery having proximal and distal ends. The probe is selectively advanceable within the cannula to expose the distal end of the probe from the distal end of the cannula. A library is also included having a plurality of overlays. Each overlay includes an image depicting a treatment profile for a particular probe. The treatment profile estimates a depth of therapeutic treatment upon activation of the probe.

The image of each overlay depicts a particular thermal profile which surrounds the exposed distal end of the probe. In one embodiment, the overlay desirably is a digital representation which can be scaled according to the size of the cannula.

In another embodiment, the system includes an imaging system for imaging the target surgical site. The imaging system includes a monitor for displaying the image of the target surgical site and is configured to operatively communicate with the library of overlays to allow selective superimposed imaging of a particular profile over the probe. Desirably, each overlay is superimposable on the image of the target surgical site.

The probe is adapted to be connected to a power source which is selectively adjustable to vary at least one operative setting. The operative settings may include temperature, impedance, RF power, RF current, RF voltage, mode of operation and/or duration of application.

In another embodiment, the system includes one or more overlays corresponding to the relative overlay exposure of the distal end of the probe from the distal end of the cannula. Additional overlays may include overlays for each operative setting of the power source.

According to another aspect of the present disclosure, a system for thermally treating target tissue having a graphical user interface is provided. The system includes a surgical device connected to a surgical generator, an imaging device displaying an image of the surgical device in situ and a database module connected to a library of thermal images. The system also includes a querying algorithm configured to select simulated thermal images. The thermal images are selected according to a desired thermal image for the surgical device based on at least one electrical settings, e.g., tip configuration of the surgical device; depth of the penetration of the surgical device; activation time of the surgical device; and combinations thereof. The system further includes a graphical user interface module configured to overlay a selected simulated image on the imaging device over the surgical device in situ.

In another embodiment, the system may include a medical image which may be a digital representation of a real time image or an archived image. The surgical device described in the system may be a probe, e.g., an electrode, microwave antenna, optical fiber and cryoablation probe. The probe may further include proximal and distal ends, the distal end of the probe being selectively advanceable to expose the distal end of the probe from the distal end of the cannula. The library of thermal images in the system may include images of an actual thermal profile of the surgical device and/or a thermal profile derived from computer simulating techniques. The querying algorithm may be configured to locate, orient and scale the thermal images according to the surgical device in situ. The graphical user interface may consist of an electronic pointing device, which facilitates identification, manipulation and/or highlighting of the medical image of the surgical device in situ. The graphical user interface may include a monitor, keyboard and electronic pointing device. The system may further include an image system which images the target surgical site on a display to operatively associate the surgical site with the library of thermal images to allow selectable positioning of a thermal image within the target surgical site.

A method of creating an overlay for performing surgical procedures is also disclosed. The method includes the steps of: providing a thermal acquisition system having a bath containing a quantity of a test gel; at least one sheet of a thermally reactive paper; a probe which is connectable to a power source and capable of delivering energy; and an image/data acquisition system operatively couplable to the power source and directed toward the bath.

The method further includes the steps of: stabilizing the temperature of the bath; placing a piece of the thermally reactive paper into the bath; placing the probe into the bath such that the probe is disposed between the thermally reactive paper and the image/data acquisition system; activating the source of power; and recording the image created on the thermally reactive paper and the parameters associated with the power source with the image/data acquisition system. The parameters recorded include temperature, impedance, RF power, RF current, RF voltage, mode of operation, amount of exposure of the probe from a distal end of the cannula, and/or duration of activation of the source of power. The method may further include the step of storing the overlay having the image and the parameters in a library accessible by the user selectively.

The method may further include the step of creating a plurality of overlays by repeating the method for each parameter and recording the image and associated parameters in the liking.

According to another aspect of the present disclosure, a method of treating a target surgical site, is provided. The method includes the steps of: providing one or more overlays including an image depicting a treatment profile of a probe, the treatment profile providing an estimation of a depth of a therapeutic treatment upon activation of a probe corresponding to the probe of the respective overlay; and superimposing the overlay(s) on an image scan of the target surgical site in order to visualize the depth of the therapeutic treatment deliverable with a probe configured according to the treatment profile of the respective overlay.

The method may further include the step of: providing a plurality of overlays, each overlay depicting a treatment profile corresponding to one of a plurality of unique probe configurations and intensity settings. The method may also include the step of providing a probe capable of delivering energy. The probe is selectively advanceable within a cannula to expose a distal end of the probe from a distal end of the cannula. The method further includes the step of providing a source of electrosurgical energy connectable to the probe.

The method may further include the steps of: imaging the target surgical site; and superimposing at least one of the overlays on the image of the target surgical site. The method may also include the step of selecting an overlay depicting a treatment profile corresponding to the therapeutic treatment and resulting effect desired.

In one particular embodiment, the method further includes the steps of: introducing the probe into the target surgical site according to the treatment profile of the selected overlay; and activating the probe according to the treatment profile of the selected overlay.

According to another aspect of the present disclosure, a method of treating a target surgical site having a graphical user interface, is provided. The method includes the initial steps of: selecting a surgical device adapted to connect to a surgical generator; displaying an image of the surgical device in situ; and connecting to a database module adapted to connect to a library of thermal images. The method further includes the steps of: querying simulated thermal images according to a desired thermal image profile for the surgical device based on one or more electrical settings of the generator, e.g., tip configuration, depth of penetration, activation time and combination thereof; and superimposing a selected simulated image atop the image of the surgical device and displaying both the image of the surgical device in situ and the simulated image on an imaging device over the surgical device in situ.

The method may further include the step of identifying, manipulating and highlighting a simulated image on the imaging device. Also, the method may include the step of querying computer generated predicted overlays according to at least one of the size, shape and type of procedure and surgical device selected.

This disclosure discloses and claims systems for thermally treating target tissue and also discloses, but does not claim, associated methods.

These and other aspects and advantages of the disclosure will become apparent from the following detailed description and the accompanying drawings, which illustrate by way of example the features of the disclosure.

### BRIEF DESCRIPTION OF THE DRAWINGS

The features of the system and method of the present disclosure will become more readily apparent and may be better understood by referring to the following detailed descriptions of illustrative embodiments of the present disclosure, taken in conjunction with the accompanying drawings, wherein:
FIG. 1 is a cross-sectional view of an intervertebral disc with a portion of the intervertebral apparatus of the present disclosure inserted into the intervertebral disc;
FIGS. 2a, 2b, and 2c show a system of components for the intervertebral apparatus of FIG. 1 for RF intervertebral disc heating or any other RF heating, thermal ablation, or cryogenic denervation, the apparatus including a cannula, impedance stylet, and electrode;
FIG. 3 is a flow chart illustrating a method of creating a database of thermal profile overlays;
FIG. 4 is a schematic view of a thermal acquisition system for creating a thermal profile overlay in accordance with the present disclosure;
FIG. 5 is an enlarged schematic illustration depicting the creation of a thermal profile image;
FIG. 6 is an exemplary thermal profile image produced by the thermal acquisition system of FIG. 4;
FIG. 7 is a schematic illustration of a system for performing surgical procedures using thermal profiling;
FIG. 8 is a flow chart illustrating an exemplary methods of performing surgical procedures using thermally profiled electrodes;
FIG. 9 is a schematic illustration of a step of the method of FIG. 8;
FIG. 10 is a schematic illustration of another step of the method of FIG. 8;
FIG. 11 is a schematic illustration of yet another step of the method of FIG. 8;
FIG. 12 is an enlarged schematic illustration of the indicated area of FIG. 11;
FIG. 13 is a fluoroscopic image of a spine illustrating a spinal needle being inserted from the left into the nucleus pulposus of a vertebral disc, and an introducer cannula and electrode being inserted from the right into the annulus fibrosus of the vertebral disc;
FIG. 14 is a fluoroscopic image of the spine of FIG. 13 illustrating an overlay, in accordance with the present disclosure, superimposed over the electrode to provide visualization of the predicted area of thermal effect;
FIG. 15 is an enlarged fluoroscopic image illustrating the electrode/thermal profile of the overlay of FIG. 14;
FIG. 16 is an overlay illustrating the tissue histology together with the actual thermal effects produced by the treatment;
FIG. 17 is a schematic illustration of a system for performing surgical procedures using thermal profiling having a graphical user interface;
FIG. 18 is a schematic illustration of a system for thermally treating target tissue having a graphical user interface which analyzes data based on physician-user outlined area on the target site;
FIG. 19 is a flow chart illustrating a method of performing a surgical treatment with a user interface combined with a querying algorithm and recommender algorithm;
FIG. 20 is an illustration of a generator with a graphical user interface; and
FIG. 21 is an illustration of a surgical instrument with a graphical user interface.

### DETAILED DESCRIPTION

The systems and methods of the present disclosure provide for a more precise controlled positioning of a thermal probe in an intervertebral disc targeted for treatment. Moreover, the systems and methods of the present disclosure provide for an improved ability to predict and/or visualize the depth of treatment possible by the thermal probe when set to various operative parameters.

It will be readily apparent to a person skilled in the art that the systems and methods of use of the systems can be used to treat/destroy body tissues in any body cavity or tissue locations that are accessible by percutaneous or endoscopic catheters or open surgical techniques, and is not limited to the disc and/or spinal area. Applications of the systems and methods in all of these organs and tissues are intended to be included within the scope of the present disclosure.

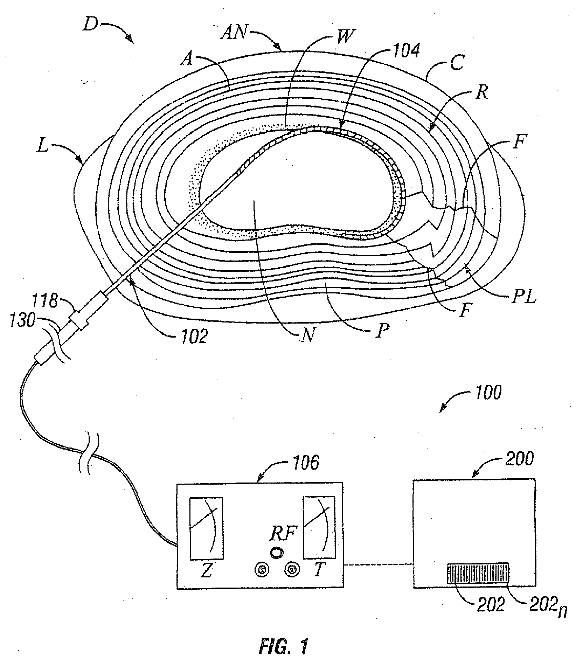

Prior to a detailed discussion of the system and methods of use of the systems and method of the present disclosure, a brief overview of the anatomy of the intervertebral disc is presented. With reference to FIG. 1, an intervertebral disc "D" is comprised of an annulus fibrosis "A" and a nucleus pulposus "N" disposed within annulus fibrosis "A". Annulus fibrosis "A" includes a tough fibrous material which is arranged to define a plurality of annular cartilaginous rings "R" forming the natural striata of the annulus. Nucleus pulposus "N" consists primarily of an amorphous gel having a softer consistency than annulus fibrosis "A". Nucleus pulposus "N" usually contains 70% - 90% water by weight and mechanically functions similar to an incompressible hydrostatic material. The juncture or transition area of the annulus fibrosis "A" and nucleus pulposus "N" generally defines, for discussion purposes, an inner wall "W" of annulus fibrosis "A". Disc cortex "C" surrounds annulus fibrosis "A", The posterior, anterior and lateral aspects of intervertebral disc "D" are identified as "P", "AN" and "L", respectively, with the opposed posterior-lateral aspects identified as "PL".

When mechanical stress is put upon an intervertebral disc or when an intervertebral disc degenerates with age, fissures, (illustrated by cracks "F" in FIG. 1), may occur in the posterior or posterior/lateral portions of the disc "D". Problems with the nerves, fissures "F" and degenerative discs can give rise to various patient problems, such as back or leg pain originating from the irritation or occurrence of these abnormalities. Moreover, these conditions may ultimately result in conditions such as bulging or herniated discs. Heating and/or electromagnetic field (EMF) therapy of intervertebral disc "D", for example, annulus fibrosis "A" in the posterior "P" or posterior-lateral "PL" portions, will result in denervation of nerves and/or alterations and thermal ablation of disc structures, which will, in turn, produce alleviation of pain and healing of the disc. Thus, it is desirable, to insert and place a thermal or electromagnetic probe in posterior "P" and/or posterior-lateral "PL" portion of intervertebral disc "D" where these neural and aberrant structures occur for the relief of pain and other disc related problems.

### 1. System for Thermally Profiling Surgical Electrode

In the drawings and in the description which follows, the term "proximal", as is traditional, will refer to the end of the system, or component thereof, which is closest to the operator, and the term "distal" will refer to the end of the system, or component thereof, which is more remote from the operator.

With reference to FIG. 1, in accordance with an embodiment of the present disclosure, a system of using RF energy and thermal profiling in surgical procedures is generally designated as 100. System 100 includes an outer insertion or introducer cannula 102, a probe for energy delivery (e.g., electrode, thermal probe, EMF probe, electrosurgical probe, etc.) 104 which is positionable within cannula 102, an electrosurgical generator, power source or the like 106 connected to probe 104. Optionally, system 100 can include an impedance stylet 108 which is also positionable within cannula 102.

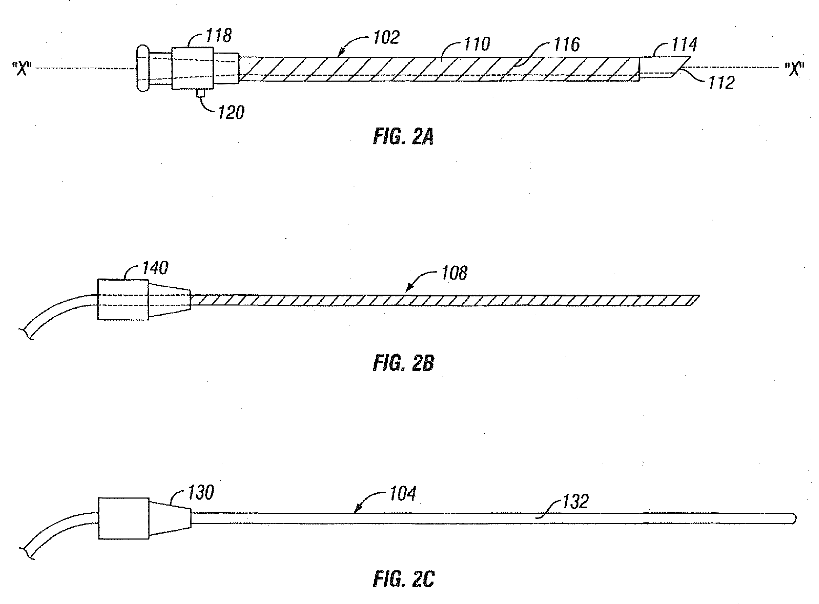

As seen in FIGS. 1 and 2a, introducer cannula 102, typically is a rigid tubular shaft 110 defining a longitudinal axis "X". Tubular shaft 110 which may include a beveled tip 112 adjacent the distal end 114 and angled with respect to the longitudinal "X" axis. Beveled tip 112 may be angled from about 15° to about 45°. Shaft 110 may be composed of a conductive material such as stainless steel or other suitable composition and is insulated with insulation 116 along at least a portion, of the length thereof. Alternatively, shaft 110 may be fabricated from a suitable polymeric material and formed by conventional injection molding techniques. Distal end 114 of shaft 110 may be left un-insulated or exposed to allow electrical communication with the tissue as cannula 102 is placed in the tissue. (e.g., for impedance measuring, etc.) A handle or housing 118 is connected to a proximal end of cannula 102 and may include an index marker 120 to indicate the direction of beveled tip 112 such that when probe 104 is introduced within cannula 102, the surgeon may determine in which azimuthal rotational direction beveled tip 112 is oriented.

Shaft 110 may have a diameter ranging from a fraction of a millimeter to several millimeters and a length of a few centimeters up to about 20 centimeters or more. Alternatively, shaft 110 may be fabricated from an MRI (Magnetic Resonance Imaging) compatible material, including cobalt alloys, titanium, copper, Nitinol, etc.

Power source or generator 106 may be, for example, a radiofrequency generator providing energy at frequencies between several kilohertz to several hundred megahertz. Generator 106 may have a power output ranging from several watts to several hundred watts, depending on the clinical need. Generator 106 typically includes control devices to increase or modulate power output as well as readout and display devices to monitor energy parameters such as voltage, current, power, frequency, temperature, impedance, etc., as appreciated by one skilled in the art. Other types of power sources and/or generators are contemplated, e.g., including and not limited to resistive heating units, laser sources, or microwave generators.

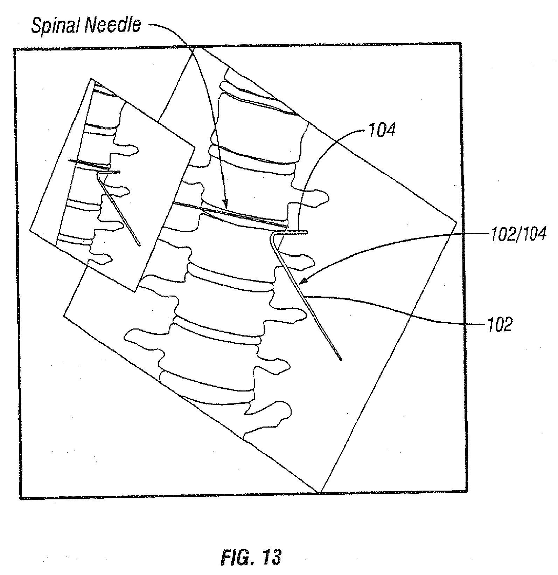

With continued reference to FIGS. 1 and 2a-2c, probe (e.g., thermal or EMF probe) 104 of system 100 will be discussed. As seen in FIGS. 1 and 2c, electrode 104 is positionable within cannula 102 and is adapted for reciprocal movement therewithin. When used as a radiofrequency probe, probe 104 is a monopolar system and is used in conjunction with an extended surface area grounding pad 134 (see FIG. 13) which contacts the patient's skin over a very large surface area relative to the exposed surface area of the electrode tip. In addition, when used as a radiofrequency probe, electrode 104 may be insulated except for a distal portion thereof which may be left un-insulated for transmission of energy. Alternatively, and in one particular embodiment, probe 104 may be entirely un-insulated while cannula 102 functions as the insulating element of the apparatus. In this arrangement, the degree of extension of the distal end portion of probe 104 beyond beveled tip 112 determines the heating capability of electrode 104. Probe 104 includes a handle 130 and an elongated member or rod 132 extending distally from handle 130. An exemplary embodiment of a thermal or EMF probe is provided in U.S. Patent 6,604,003 to Fredricks et al., the entire contents of which is incorporated herein by reference.

As seen in FIGS. 1 and 2b, impedance stylet 108 is positionable within the lumen of cannula 102 and occludes the front opening of cannula 102 to prevent entry of tissue, fluids, etc., during introduction of cannula 102 within intervertebral disc "D". Stylet 108 may include a proximally positioned hub 140 which mates with housing 118 of cannula 102 into which stylet 108 is introduced to monitor impedance of the tissue adjacent the distal end of cannula 102. Once the combination of stylet 108 and cannula 102 are inserted into the body, impedance monitoring assists in determining the position of beveled tip 112 of cannula 102 with respect to the patient's skin, cortex "C", annulus fibrosis "A", and/or nucleus "N" of intervertebral disc "D". Each of these regions will have different impedance levels which are readily quantifiable.

For example, for a fully insulated electrode or cannula with an exposed area of a few millimeters at the cannula end, the impedance will change significantly from the position of the tip near to or contacting cortex "C" of intervertebral disc "D" to the region where the tip is within annulus fibrosis "A" and further where the tip is within nucleus "N" of intervertebral disc "D". Differences in impedance can range from a few hundred ohms outside intervertebral disc "D", to 200 to 300 ohms in annulus fibrosis "A", to approximately 100 to 200 ohms in nucleus "N".

This variation can be detected by the surgeon by visualizing impedance on meters or by hearing an audio tone whose frequency is proportional to impedance. Such a tone can be generated by a monitor (not shown). In this way, an independent means is provided for detecting placement of cannula 102 within intervertebral disc "D". Thus, for example, in an application where an electrode 104 in the form of an EMF probe is to be inserted between adjacent layers of annular tissue, undesired penetration of the tip of EMF probe 104, extending from cannula 102, through inner wall "W" of annulus "A" and into nucleus pulposus "N" can be detected via the impedance monitoring means.

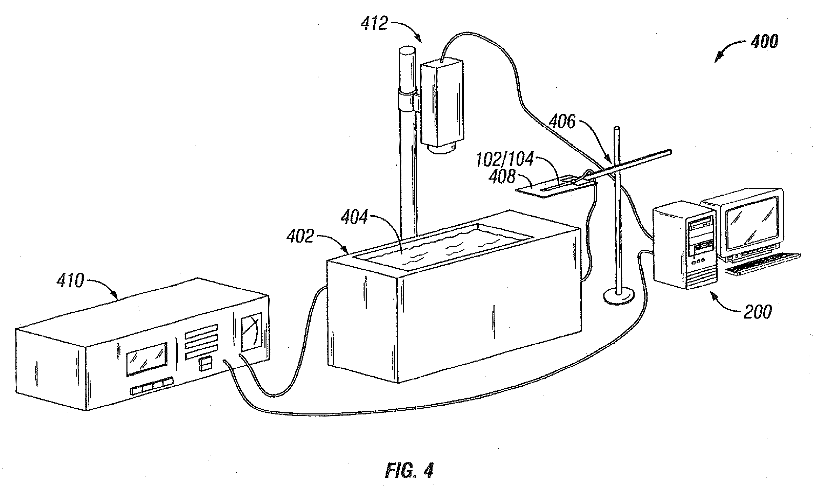

As seen in FIGS. 1, 4 and 7, system 100 further includes a library 200 including a plurality of thermal profiles/overlays 202. As used herein, the term library is understood to include and is not limited to repository, databank, database, cache, storage unit and the like. Each overlay 202 includes a thermal profile which is characteristic of and/or specific to a particular configuration of cannula/electrode assembly or amount of exposure (i.e., specific to the amount of probe 104 extending from the distal tip of cannula 102) of the cannula/electrode assembly. In addition, for each amount of exposure or configuration of the cannula/electrode assembly, a plurality of overlays 202 is provided which includes a thermal profile which relates to, for example, the amount of time probe 104 is activated, the temperature to which probe 104 is heated, the frequency of the probe, etc.

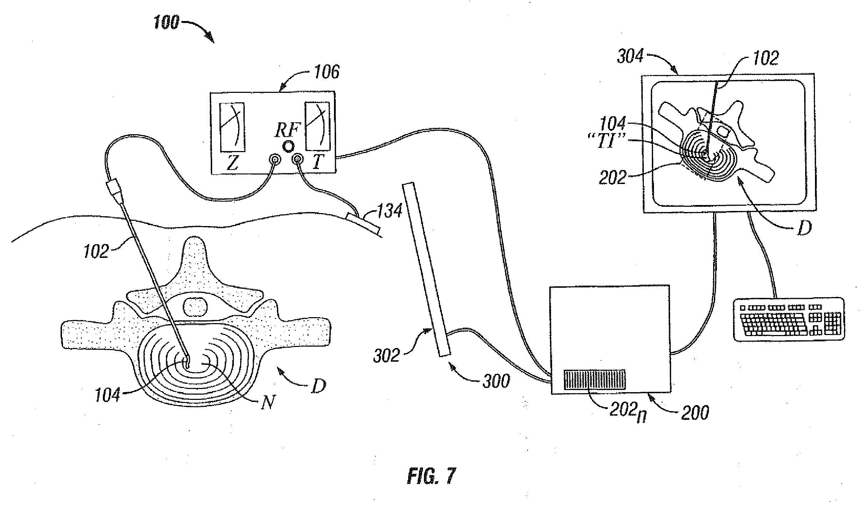

As seen in FIG. 7, system 100 further includes an imaging system 300 configured and adapted to image and display a target surgical site. Imaging system 300 includes an imaging device 302, in the form of an x-ray imager, a CT scanner, an MRI device, a fluoroscopic imager and the like, and a monitor 304 for displaying the image produced by imaging device 302. The library 200 is configured to operatively communicate with imaging system 300.

### 2. Method of Creating Thermal Overlay

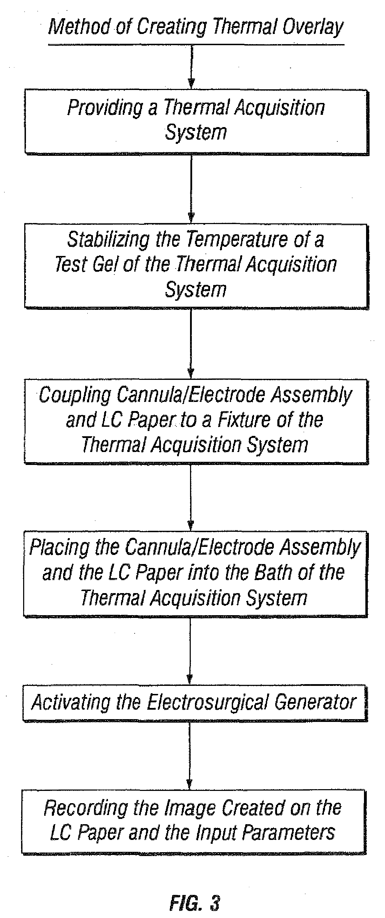

Turning now to FIGS. 3-6, a method of creating a thermal overlay 202 (of a plurality of thermal overlays 202), is illustrated and described. Creation of a thermal overlay 202 includes the initial step of providing an acquisition system 400, which may be a thermal acquisition system. Thermal acquisition system 400 includes a bath 402 containing a quantity of a transparent test gel 404 (e.g., SMK/RFK formulation conductive polymer), a fixture 406 configured and adapted to support a cannula/ probe assembly 102/104 and a piece of thermally reactive paper, for example, thermal liquid crystal (LC) paper 408. The system also includes an electrosurgical generator 410 operatively connected to cannula/electrode assembly 102/104, and an image/data acquisition system 412 operatively connected to electrosurgical generator 410 and oriented toward bath 402.

The method of creating thermal overlay 202 further includes the steps of:
- stabilizing the temperature of test gel 404 in bath 402 to approximately 30°C;
- coupling cannula/probe assembly 102/104 and LC paper 408 to fixture 406 such that cannula/probe assembly 102/104 and LC paper 408 are placed in close proximity to one another, at a predetermined distance;
- placing (e.g., submerging) cannula/probe assembly 102/104 and LC paper 408 into bath 402 such that cannula/probe assembly 102/104 is disposed between LC paper 408 and image/data acquisition system 412;
- setting electrosurgical generator 410 to a predetermined setting "lesion" or continuous mode at a temperature of about 42°C or about 80°C;
- activating and/or stimulating electrosurgical generator 410 such that thermal radiation emanating from probe 104 impinges LC paper 408 to create a thermal image "TT"; and
- recording, with image/data acquisition system 412, the image (i.e., temperature gradients or "halos" 150 around cannula/probe assembly 102/104) created on LC paper 408 and recording the input parameters (e.g., temperature, impedance, RF power, RF current, RF voltage, mode of operation, exposure of probe 104 from distal end of cannula 102, duration of application of the electrosurgical energy, etc.) associated with the creation of the image on LC paper 408.

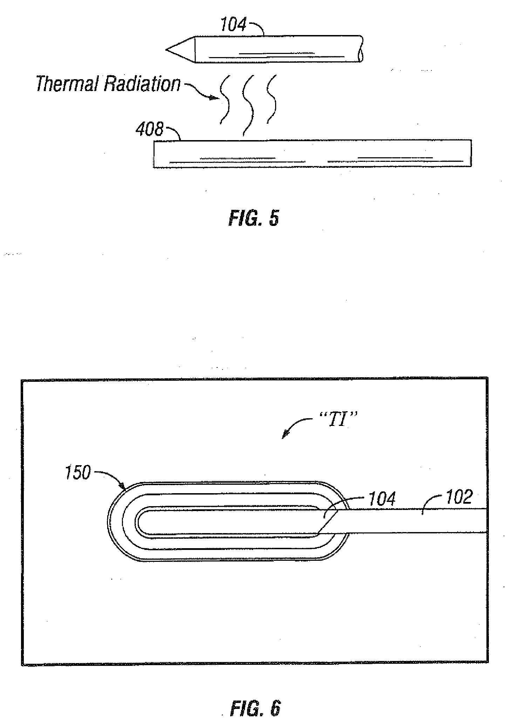

As can be appreciated from FIG. 5, temperature gradients or "halos" 150 formed on LC paper 408 include a plurality of "halos" 150 of differing color with each color representing a different temperature. The temperature at which electrosurgical generator 410 is set will determine the LC paper that is used, for example, for a temperature setting of 42°C, LC paper having a range of 35-40°C is used and for a temperature setting of 80°C LC paper having a range of 55-60°C is used. A thermal imaging camera or the like is used to record the temperature gradients produced on LC paper 408.

Thermal image "TI" and the data provided by thermal image "TI" are recorded digitally. Accordingly, the method of creating one or more thermal overlays 202 can further include the step of storing, for example, digitally, the image and the data in library 200.

The process is repeated to create an overlay 202 for each configuration of cannula/probe assembly 102/104 and each setting. In this manner, a plurality of overlays 202 is created and stored in library 200. For example, a series of overlays 200 can be created for each temperature setting of electrosurgical generator 410 (e.g., 42°C and 80°C). For each temperature setting of electrosurgical generator 410, a series of overlays 200 can be created for each tip exposure dimension (e.g., 3, 4 and 6mm) of cannula 102. For each exposure dimension of cannula 102, a series of overlays 200 can be created for each offset position of probe 104 relative to cannula 102. Offset positions are referenced to the "flush" condition (i.e., 0 mm) which is obtained by placing a flat surface flush against the bevel of cannula 102 and inserting probe 104 into cannula 102 until probe 104 contacts the flat surface.

As seen in FIG. 6, the shapes of thermal images "TI" are usually elliptical and are centered on the exposed tip of probe 104. The major axis of the ellipse can be measured using an image analysis software program. Thermal image "TI" is represented by a series of gradations or rings 150, each ring 150 representing a different temperature intensity.

Creation of the thermal overlays according to the present method provides visual information that will assist in comparing the performance between different electrodes and different electrosurgical generators.

While the above-described method is a particular method of creating a thermal overlay, it is envisioned that other methods are also possible. For example, it is envisioned that a thermally responsive gel or paint (e.g., a composition containing quantities of a thermally responsive substance therein) may be applied to the surface of a sample tissue (e.g., human cadaver tissue, porcine tissue and the like). The cannula/probe assembly 102/104 may then by introduced into the sample tissue and electrosurgical generator 410 activated in accordance with the method described above in order to create a thermal profile on the surface of the sample tissue. The thermal profile may be recorded in a manner similar to the method described above. This procedure may be repeated as many times as necessary in order to produce thermal profiles for various insertion depths of cannula/probe assembly 102/104 into the sample tissue, for various settings of electrosurgical generator 410, and/or for various configurations of cannula/probe assembly 102/104. In this manner, the effects of cannula/probe assembly 102/104 may be easily mapped on tissue.

### 3. Method of Performing Surgical Procedures

Prior to a detailed discussion of the methods of performing surgical procedures in accordance with the present disclosure, a brief overview of a general method of performing thermal treatment of an intervertebral disc is discussed. With reference to FIG. 1, the targeted intervertebral disc "D" is identified during a preoperative phase of surgery. Access to the intervertebral disc area is then ascertained, through percutaneous techniques or, less desirably, through open surgical techniques. Cannula 102, with stylet 108 positioned and secured therein, is introduced within intervertebral disc "D" from a posterior or posterior-lateral location. Alternatively, cannula 102 may be utilized without stylet 108.

During introduction of the cannula/stylet assembly 102/108, the impedance of the tissue adjacent the distal end of cannula 102 is monitored. Impedance monitoring may be utilized to determine the position of the tip of cannula 102 with respect to the patient's skin, the cortex "C", the annulus fibrosis "A" and/or the nucleus pulposus "N" of the intervertebral disc "D". As discussed above, these regions have different and quantifiable impedance levels thereby providing an indication to the user of the position of the tip of cannula 102 in the tissue. Monitoring of the location of the tip of cannula 102 may also be conformed with use of imaging system 300. Typically, the tip of cannula 102 is positioned within annulus fibrosis "A" of intervertebral disc "D" at a posterior lateral "PL" location of intervertebral disc "D" without penetrating through inner wall "W" and into nucleus "N".

With cannula 102 in the desired position, stylet 108 is removed and probe 104 is positioned within cannula 102 and advanced therethrough. Probe 104 is advanced an amount sufficient to at least partially expose a distal portion thereof from the tip of cannula 102. The degree of exposure of the distal end portion of probe 104 from the tip of cannula 102 may be indicated by distance or indexing markings provided on rod 132 of probe 104.

Once probe 104 is positioned within annulus fibrosis "A" as desired, power source 106 is activated whereby probe 104 delivers thermal energy and/or creates an electromagnetic field adjacent intervertebral disc "D" to produce the thermal and/or EMF therapy desired. Appropriate amounts of power, current, or thermal heat may be monitored from power source 106 and delivered for a certain amount of time as determined appropriate for clinical needs. For example, if denervation of nerves surrounding intervertebral disc "D" is the objective, the tissue adjacent the exposed end of probe 104 is heated to a temperature from about 45°C to about 60°C. If healing of fissures in intervertebral disc "D" is the surgical objective, the temperature in the tissue is raised to about 60°C-75°C.

As can be appreciated by one of skill in the art, the degree and/or amount of exposure of the distal portion of probe 104 from the tip of cannula 102 controls the volume of disc tissue heated by probe 104. Sensors (not shown) can be used to provide information concerning the temperature of tissue adjacent probe 104. Alternatively, impedance means (not shown), associated with, e.g., probe 104, can provide impedance measurements of the tissue thereby providing an indication of the degree of desiccation, power rise or charring, that may be taking place near the exposed distal portion of probe 104. This indicates the effectiveness of the treatment.

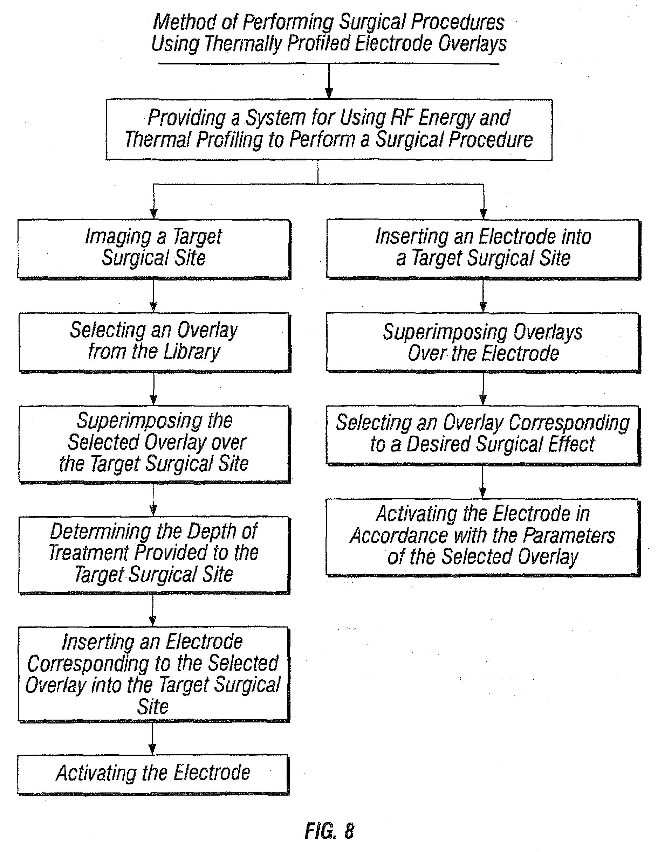

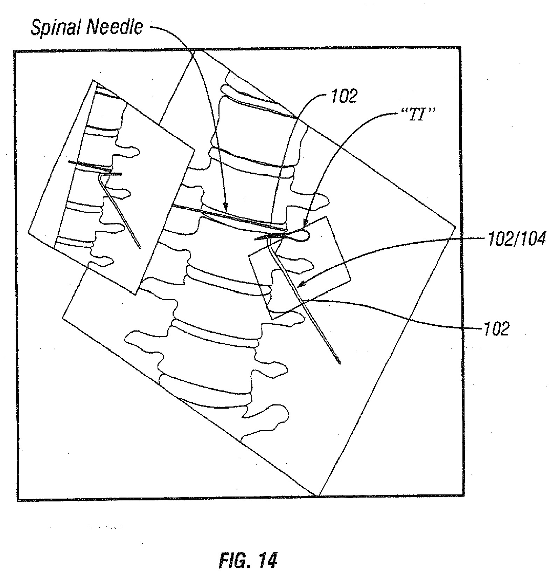

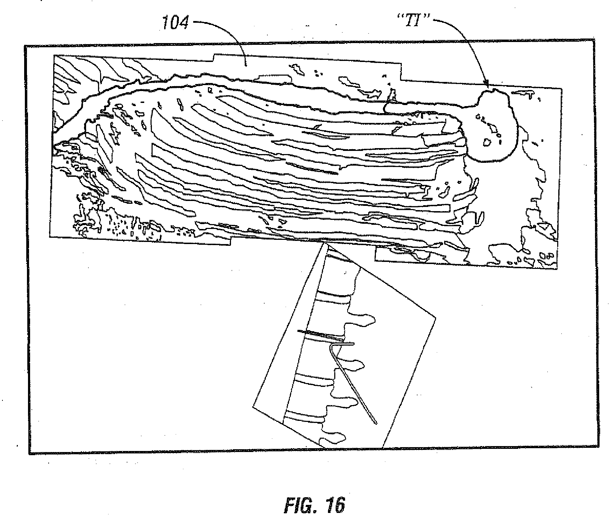

Turning now to FIGS. 7-16, in accordance with the present disclosure, a method of performing a surgical procedure using thermally profiled electrode overlays is illustrated and described. The method includes the initial step of providing a system 100 for using RF energy and thermal profiling in surgical procedures. As described above, system 100 includes an introducer cannula 102, at least one probe 104 positionable within cannula 102, a library 200 including a plurality of thermal overlays 202. An imaging system 300 is also included which is configured and adapted to take images of a target surgical site and display the images of the target surgical site to the operator.

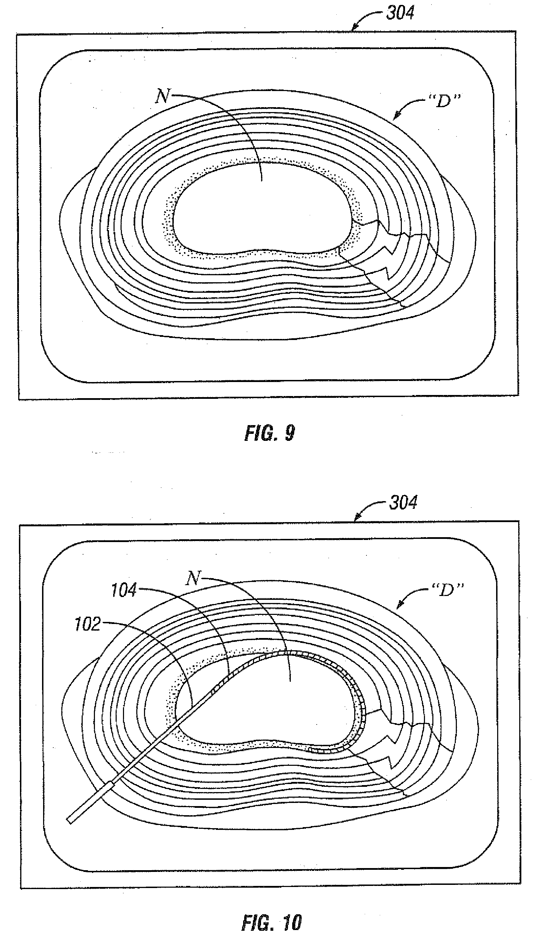

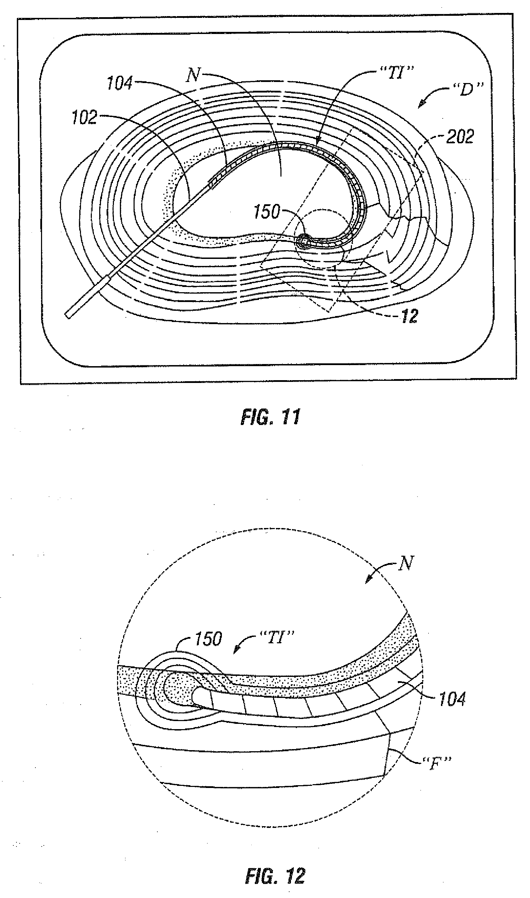

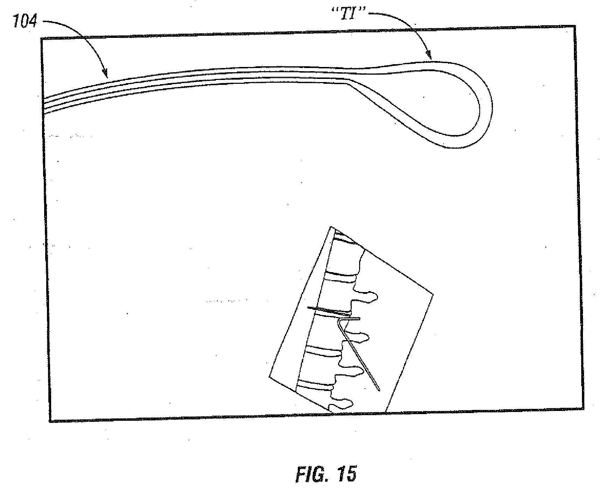

The method of performing the surgical procedure further includes the steps of:
- imaging the target surgical site with imaging system 300 in order to display the target surgical site on monitor 304, see FIGS. 7 and 10;
- selecting an overlay 202 from the plurality of overlays 202 stored in library 200 relating to the desired treatment effect of a particularly shaped probe;
- superimposing the selected overlay 202 over the imaged target surgical site, see FIGS. 7, 11, 12, 14 and 15;
- evaluating the scope, degree and/or depth of treatment provided to the target surgical site by using and/or configuring system 100 to the parameters corresponding to and/or associated with the selected overlay 202;
- inserting a cannula/probe assembly 102/104, including a probe 104 corresponding to the electrode parameters of the selected overlay 202, into the target surgical site, see FIGS. 11 and 15; and
- activating and/or stimulating probe 104 according to the parameters corresponding to and/or associated with the selected overlay 202.

In an alternative method, probe 104 is inserted into the target surgical site (see FIGS. 11 and 13) prior to superimposing overlay 202 thereon. With probe 104 in position, various overlays 202 are superimposed over probe 104 in order to illustrate the various depths of thermal penetration possible and in order to determine the desired and/or appropriate operative and/or activation parameters for probe 104, see FIGS. 12, 14 and 15. An overlay 200 is selected which corresponds to a surgical effect desired. Probe 104 is then activated in accordance with the parameters of selected overlay 202.

In either of these methods, the selected overlay 202 provides the operator with a visual representation of the depth of thermal penetration produced by probe 104 when set to the parameters of the selected overlay 202. In addition, the selected overlay 202 enables the operator to better visualize the desired placement of probe 104 and/or enables the operator to guide probe 104 into the target surgical site along a path corresponding to the direction of the thermal profile of the selected overlay 202. Moreover, the thermal visualizations offered by overlays 202 can assist in identifying mechanisms of action and optimizing the desired effects. In addition, the operator may compare the various effects of a variety of differently-shaped electrodes to optimize surgical outcome.

FIGS. 13-16 are fluoroscopic images of the spine illustrating the steps of the methods described above.

The implementation and use of overlays 202 on monitors 304 can range from simple systems where the operator manually places overlay 202 on monitor 304 or to more sophisticates pattern recognition systems. The pattern recognition systems could be used to identify the treatment parameters selected by the operator, select the appropriate overlay 202 from library 200, and project and/or display overlay 202, at appropriate scale and placement, on monitor 304. As can be appreciated, this enables the surgeon to visualize and estimate the and result of the treatment and overall tissue effect (e.g., thermal spread) before energizing the electrode.

### 4. System for thermally treating target tissue having a graphical user interface

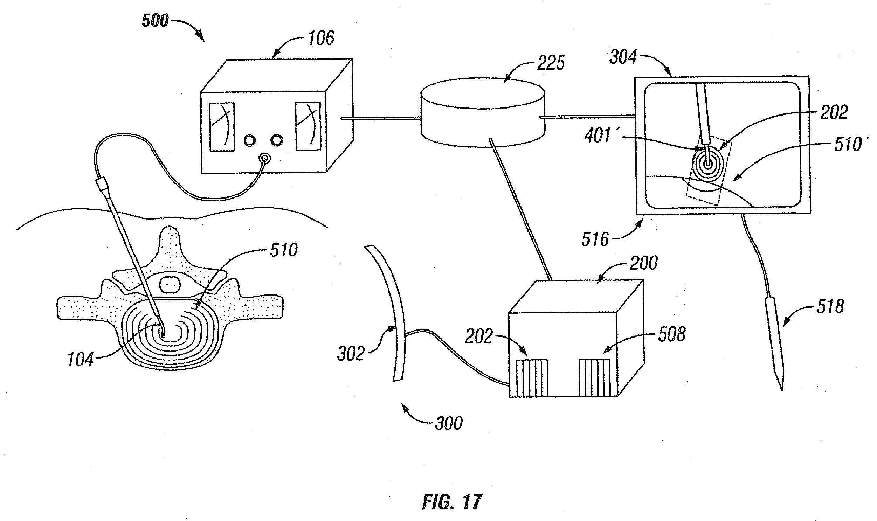

FIG. 17 illustrates another embodiment according to the present disclosure which includes a system 500 for thermally treating target tissue having a graphical user interface. More particularly, system 500 includes a surgical device 104 adapted to connect to a power source (e.g., surgical generator 106) and an imaging device 302 which displays an image 510' of the surgical device in situ (at the target surgical site) 510. A database module 225 is operatively coupled to a library 200 of thermal overlays 202 or a plurality of computer-simulated thermal overlays 508. For the purposes herein, the thermal overlays 202 are actual overlays created by the aforementioned method utilizing thermal paper and digital photography wherein computer-simulated overlays are overlays which may be manipulated by the user based on different surgical parameters, tip configurations, depth of penetration, different surgical techniques, previously-recorded surgeries, etc.

A querying algorithm 502 (not shown) may be included which is configured to enable a user to select one or more simulated thermal overlays 202 or computer simulated thermal overlays 508 according to a desired thermal image for the surgical device 104 used in a particular surgery or for a particular surgical purpose. For example, the querying algorithm 502 enables a user to select one or more thermal overlays 202 or computer simulated thermal overlays 508 based on various electrical settings of the power source 106, tip configuration of the surgical device 104, depth of penetration of the surgical device 104, activation time of the surgical device 104 and combinations thereof. A graphical user interface system 516 (e.g., a monitor 304) is connected to the system 500 to facilitate selection of various thermal or computer-simulated overlays 202 and 508, respectively. It is envisioned that graphical user interface or monitor 516 may be connected to a group of devices which include a power source 106, image display 304 and querying algorithm 502.

It is also envisioned that a particular medical image 510' may be selected from a group (not shown) consisting of real time images and archived images of a particular patient or surgery. Real time images are images that appear live, whereas archived images are pre-recorded. It is also envisioned that archived images may be a digital representation, for example DICOM format, which may be stored in a library or the like. A physician-user may retrieve an archived medical images, for example similar to their patient or study, and simulate a treatment plan. Archived medical images may be stored in database modules, for example archival systems such as PACS or the like.

As seen in FIG. 17, system 500 further includes imaging system 300 configured and adapted to display a surgical device in situ 510' as well as a selected overlay 202 or 508. Imaging system 300 may include one or a combination of the following known imaging devices 302: an x-ray imager, a CT scanner, an MRI device, a fluoroscopic imager or the like for scanning or reading a surgical site 510 and a monitor 304 for displaying the surgical site image 510' produced by imaging device 302. As mentioned above, library 200 is operatively coupled to imaging system 300 to allow a surgeon to superimpose overlays 202 or 508 on a monitor 304.

Thermal overlay 202 may be a thermal image depicting a thermal profile of the surgical device 104. It is envisioned thermal overlay 202 may have certain stored data inputs which may include input parameters for a power source 106. When the suggested input parameters of the overlay are entered, the power source 106 delivers the energy to surgical device 104 and creates the energy of the thermal overlay 202 depicted on the image display 304. A method of creating a thermal overlay 202 is described more in detail above. Alternatively (or in addition to), the surgeon may select a computer-simulated image 508 and overlay the computer-simulated image 508 atop the active tip of the surgical device to visualize "what if" scenarios when adjusting various surgical parameters such as, generator controls, depth of penetration, type of tip being utilized, etc. In one envisioned embodiment, one or more thermal images 202 are overlayed on the surgical device and displayed on the monitor and one or more computer-simulated overlays are superimposed atop the thermal images 202.

Querying algorithm 502 may be utilized to facilitate or enhance the ability of the user to search the library 200 of thermal overlays 202 or computer simulated thermal overlays 508 and locate one or more relevant overlays 202 or 508 for a particular surgical purpose. Each overlay 202 or 508 may be oriented and scaled to correspond with the scale of the surgical device in situ 510 as displayed on the monitor 304. It is envisioned that part of the querying process may be searching the library 200 of thermal overlays 202 or computer simulated thermal overlays 508 according to the type of surgical device 104 attached to system 500 or displayed on an archived image (not shown) if a surgical device 104 is not connected. Thermal overlays 202 or computer simulated thermal overlays 508 may be based on various electrical settings of the generator 106, tip configuration of the surgical device 104, depth of penetration of the surgical device 104, activation time of the surgical device 104 and combinations thereof. The querying system 502 may also be configured to determine similarities of the thermal overlays 202 or computer simulated thermal overlays 508 and the displayed image 510' displayed. Each surgical device 104 may have a different thermal 202 image and therefore the thermal overlay 202 will portray a different treatment effect in surgical site 510.

The graphical user interface or monitor 516 may include an electronic pointing device 518 to facilitate selection or manipulation of a particular overlay 202 or 508 on the displayed image 510'. Alternatively, the graphical user interface system 516 may include touch screen capabilities, a mouse, a stylet or interact with the user using voice-activated commands.

System 500 is also envisioned to include the capability of predicting the thermal treatment of a device in a computer simulation or utilizing a computer simulated overlay. In other words, the user may select a particular thermal overlay 202 and the system may be able to provide a computer-generated simulation of the thermal treatment of tissue from activation to a preset time period based on the input parameters by the user. Moreover, if the particular actual thermal image 202 for a particular instrument or tip configuration is not archived in the library based on the input parameters, the system 500 may be configured to utilized one or more computer-simulated overlays 508 to extrapolate a thermal profile of the treatment zone. For example, the querying algorithm 502 (or a separate algorithm) may be designed to locate and select a predicted computer generated overlay 508 from the library 200 of images that most closely corresponds to the target surgical site. Predicted computer generated overlay 508 may be a computer-generated image of the target surgical site after treatment. The predicted overlay 508 may be part of library 200 of a plurality of predicted overlays 508.

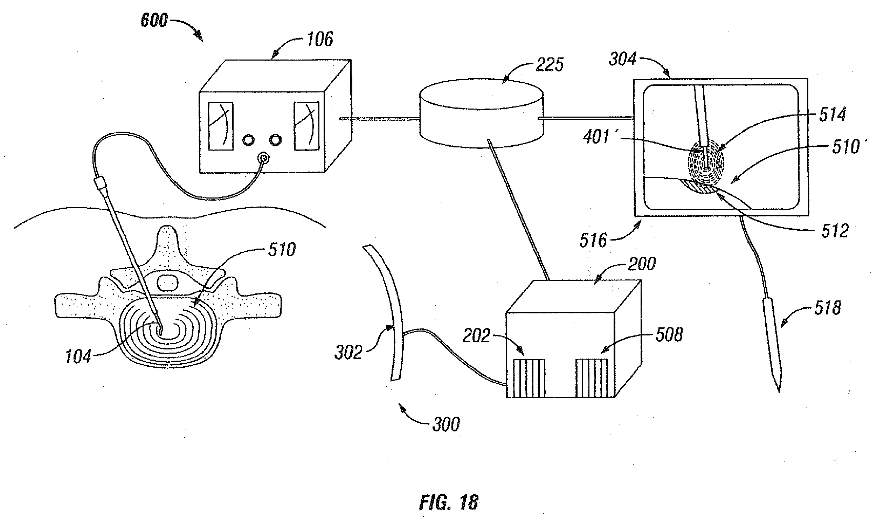

As best shown in Fig. 18, the present disclosure also relates to a system 600 and method of thermal imagery which analyzes data based on a physician-user outlined area on the target site. For example, it is envisioned that the user or physician may mark up the desired treatment zone on the monitor 304 and the querying algorithm 502 may then be activated to match the outlined treatment zone 512 with a particular surgical device, tip configuration, depth of penetration, etc. to satisfy the desired treatment zone 512 indicated by the physician.

System 600 may also include imaging tools 302, e.g., a magnetic resonance imager (MRI), which provides thermal imagery in real time or near real time on monitor 304 to allow a physician to monitor thermal surgical procedures in situ during the operation. Other types of imaging devices are also contemplated as is known in the art.

The present disclosure may also relate to a method for thermally treating target tissue utilizing a graphical user interface utilizing system 600. The method includes the initial steps of selecting a surgical device 104 adapted to connect to a surgical generator 106 and displaying the treatment site in situ 510'. The method would also include connecting to a database module 225 adapted to connect to a library 200 of thermal images 202, 508. The method also includes the steps of introducing the surgical device 104 to the treatment site 510 and querying a library 200 of thermal overlays 202, 508; superimposing a selected simulated image 514 atop the image of the surgical device 104' and displaying both the image of the surgical device in situ 104' and the simulated image 510' on monitor 304 by an imaging device 302 over the surgical device 104 in situ 510.

The method may also include superimposing one or more overlays atop the tissue to ascertain a desired treatment area 512 for the tissue based on tissue type, generator settings, type of surgical device, tip configuration of the surgical device and/or depth of penetration of the surgical device. The method also includes the step of selecting a desired overlay and configuring the generator and surgical instrument accordingly to treat tissue.

The method may also include the steps of providing an interactive device 518 such as a mouse, pencil, stylet, touch screen, voice command for identifying and outlining a desired treatment zone 512 across the tissue. The method would also include the step of utilizing the querying algorithm 502 (not shown) to select or recommend one or more surgical instruments, general settings, tip configurations and depth of penetration for achieving the desired tissue treatment.

It is also envisioned that the physician may initially choose a particular instrument, tip configuration, power setting or penetration depth and then utilize the querying algorithm to display a range or treatment options to achieve the desired treatment result, according to one or more variable, e.g.. size, shape and type of procedure and surgical device selected. If no appropriate overlay is available in the library of images 200, the querying algorithm is configured to extrapolate or scale one or more overlays 202, 508 to achieve an approximate desired result. The querying algorithm may be utilized to automatically select one or more recommended overlays 202, 508 based on the tissue type, generator setting, tip configuration, type of instrument or desired depth of penetration and display such recommendation on the monitor for interactive communication with the physician through one or more of the above interactive devices. The physician may also be able to scroll through the library 200 of images and pick a desired image 202, 508 manually if desired.

As seen in FIG. 18, it is also envisioned that the physician may utilize the overlay during the course of tissue treatment and intermittently display the overlay atop the actual treatment zone for verification purposes. The overlay may also include one or more overlay modes which allow the physician to treat the tissue without the overlay interfering with the physician's view of the treatment area. For example, a phantom mode (or ghost mode) 514, an outline mode or a strobe-like mode may be employed for this purpose which displays the overlay 202, 508 in a particular fashion (e.g., lighter contrasting color for phantom mode) so not to impede visualization of the treatment zone 512.

As can be appreciated, utilizing the overlays 202, 508 prior to activation allows a surgeon to select the most appropriate generator setting, surgical instrument, tip configuration and depth of penetration for a particularly-sized treatment area without activating of the surgical device 102. Once the physician determines whether the area to be treated is appropriately covered by the selected thermal overlay 202, 508 based on the generator settings, instrument, tip configuration and/or depth of penetration, the physician is free to activate the instrument to treat the tissue.

### 5. Method of Performing a Surgical Treatment with a User Interface

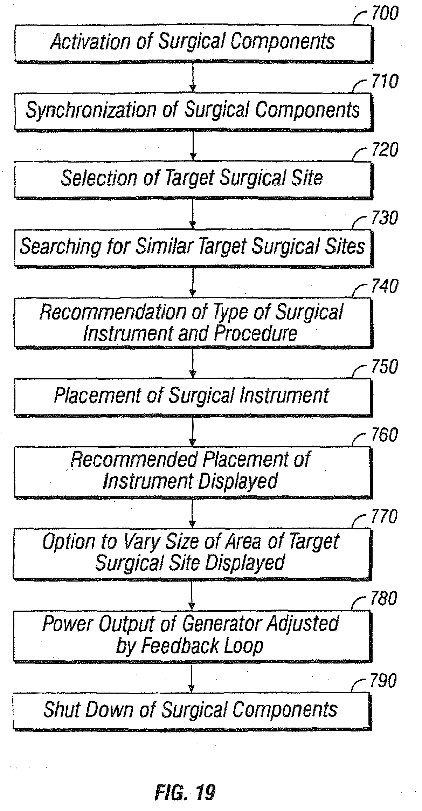

Turning now to FIG. 19, steps 700 - 790 illustrate and describe a method of performing a surgical treatment with a user interface combined with a querying algorithm and recommender algorithm. In step 700, the generator 106, user interface 516 and the surgical instrument 104 are activated. Activation may be executed manually or automatically. The user may manually activate each component by interacting with the user interface or by remote control. Alternatively, the generator 106 may be configured to activate automatically, for example, when specific or all the components of the surgical operation are connected and ready for use. A feedback loop may be used for this purpose.

In step 710, the components, for example, the imaging device, generator and surgical instrument are synchronized. Synchronization may be required for tuning the surgical instrument, for example ultrasonic surgical instruments. Synchronization may also be required for compatibility purposes since certain types of power generators and surgical instruments may need to be programmed to work together. In the event any of the components are not compatible with each other, an indicator may be provided to alert the user via an audible alarm, a visual alarm and/or an error message displayed on the graphical user interface of the generator 106.

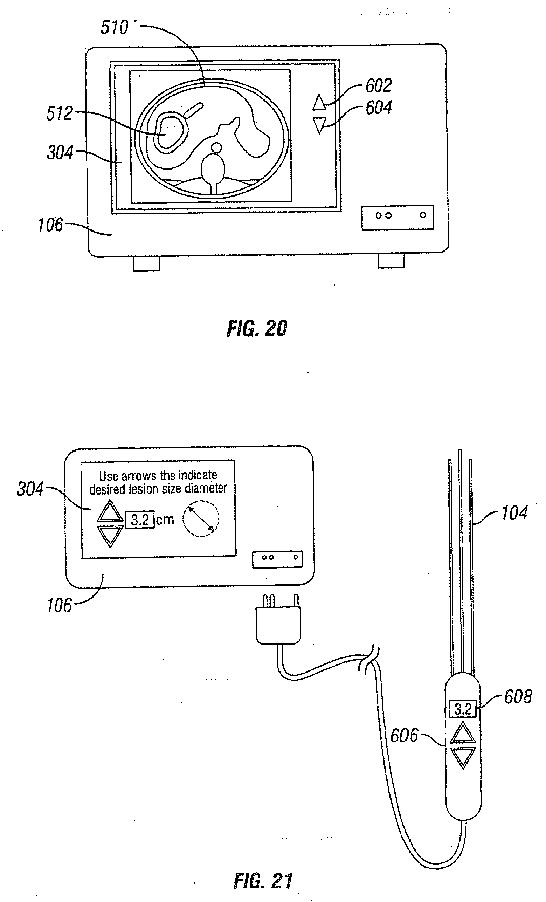

In step 720, the user views the entire surgical site 510' via screen 304 sent by an imaging device, not shown, (e.g., a CT scanner, an MRI, or the like), as illustrated in FIG. 20. The user then selects target tissue, for example a tumor, that may require ablation.

Further in step 730, the querying algorithm 502 searches the database, described in detail above, for a substantially similar stored image of a tissue volume from a library 200 as discussed above. As mentioned above, the library 200 or database 225 may contain overlays, computer generated simulations, or video of previously performed surgical procedures on similar tissue volumes.

Step 740 provides a recommender algorithm to search through the database, library or look-up tables for actual or computer-generated simulations of recommended treatments for the tumor volume based on this pre-recorded data. The treatment may include electrode shape, size, orientation, actuation levels and actuation times. In addition, the database, library or look-up table may be searched for recommended instrument types, models, and/or sizes for use with specific treatment procedures. Other examples may include, the recommender algorithm finding a video of a previous surgical procedure that used an antenna to ablate a similarly sized tumor. In this manner, the user may view the recommended instrument and procedure and compare it to a pre-planned treatment or procedure. At any time the user may follow the recommended procedure or proceed manually. The user may also decide to alter the recommended surgical procedure and adjust the settings on the user interface if applicable.

At step 750, the user places the surgical instrument recommended by the recommender algorithm or user's choice into the patient and proceeds with the surgical procedure. Further at step 760, when the user has initiated the surgical procedure an instrument locating algorithm may scan the entire surgical site and display on the graphical user interface where the surgical instrument is located. The recommender algorithm may recommend a location for the user to place the surgical instrument or a cluster of locations for placement of the surgical instrument. For example, it is contemplated that the recommender algorithm may recommend a particular ablation electrode being activated in a series of steps to properly ablate the tissue.

The recommender algorithm may create an electrosurgical protocol based on voltage, current and activation time, model, and size of the surgical instrument. The electrosurgical protocol provides for a more accurate recommendation as to which surgical instrument may be used for a particular surgical procedure.

At any one of these steps an alarm may activate if a particular surgical instrument is misplaced relative to the recommended placement area. Also, at any one of these steps an alarm may activate if healthy tissue is being affected and/or if the target tissue volume should no longer be treated, e.g., a complete alarm. Also in the present disclosure, at any time the surgical procedure may be modified and/or altered by user.

In step 770 the user interface displays an option for the user to vary the size of target surgical site area. FIGS. 20 and 21 illustrate a graphical user interface where the generator 106 and/or the surgical instrument 104 have a graphical user interface to control the size of the desired tissue volume 512.

FIG. 20 shows a generator 106 that has a touch screen 304 integrated therein which may be utilized in an interactive fashion depending on user preference. The user is able to adjust the overall size of the desired tissue volume 512, via the user interface, by using up and down arrows 602, 604 on the touch screen or possibly an interactive tool. Also, as shown in FIG. 21, the user may vary the size of the target tissue volume 512 to be treated through various input devices 606 (for example, a mouse, a pen, or a wand) that may be on the surgical instrument 104, a handheld electrode or antenna. The input device 606 may have up and down arrows to vary the size of the desired target tissue volume. The surgical instrument 104 and input device 606 may also have a display 608, e.g., an LCD, that displays the size of the target tissue volume and the desired treatment.

Alternatively, a user may adjust the size of the area to be treated by drawing or selecting the area displayed on the screen. The user interface on the generator 106 may comprise a touch screen 304 that indicates an image of the surgical site 510'. The image is produced by an imaging device (not shown), as described above. The user may indicate a target surgical site 512 where the surgical procedure should take place. The user may select the target surgical site 512 with a mouse, stylet, touch screen or the like. The recommender algorithm may be configured to utilize this information and recommend a particular instrument electrode, placement, ablation series and/or activation energy and time.

In addition, a plurality of overlays may be placed directly on the surgical site image 510' and manually compared. The group of overlays may comprise an overlay of a particular surgical instrument thermal plumes, a target surgical site, or a predicted end result target surgical site. The overlays may be displayed during surgery or for consultation of the surgical procedure.

The user proceeds with the surgical procedure (e.g., ablation, cauterization, etc.) and the graphical user interface may display thermal arrays in order to characterize the difference between temperature gradients.

In addition, the thermal temperature may be used to monitor the surgical site, e.g., the target volume. The target surgical site may be mapped out as a graph and each location would be mapped at a specific coordinate. The graphical user interface may display the surgical site in a two-dimensional array or a three-dimensional array.

At step 780, a thermal sensor feedback loop may be utilized to monitor and/or control the surgical device and/or the generator. For example, the thermal sensor feedback loop may be configured to control the energy output of the surgical instrument according to the size and location of surrounding tissue of the target surgical site. Each location on the screen may have a different temperature or appearance. The user may select or exclude certain characteristics from being monitored by the thermal sensor feedback loop (for warning/alarm purposes).

In step 790 the system systematically shuts down the surgical instrument energy power source and may be configured to provide a complete alarm. Other areas of the surgical site may also be monitored.

While the above description contains many specific examples, these specific should not be construed as limitations on the scope of the disclosure, but merely as exemplifications of particular embodiments thereof. Those skilled in the art will envision many other possible variations that are within the scope and spirit of the disclosure as defined by the claims appended hereto.

In the following, there is listed numbered items of the present disclosure corresponding to the claims as originally filed of the parent application, EP 08 012 829.1.
1. A system for thermally treating target tissue, the system comprising:
   a surgical device adapted to connect to a surgical generator;
   an imaging device operable to display an image of the surgical device in situ;
   a database module adapted to connect to a library of thermal images;
   a querying algorithm configured to select a simulated thermal image according to a desired thermal image for the surgical device based on at least one of an electrical setting of the generator, a tip configuration of the surgical device, a depth of penetration of the surgical device, an activation time of the surgical device, and combinations thereof; and
   a graphical user interface module configured to overlay the selected simulated thermal image over the image of the surgical device in situ.
2. The system according to 1, wherein the thermal image is selected from the group consisting of a real time image and an archived image.
3. The system according to 2, wherein the Thermal image is a digital representation.
4. The system according to any preceding item, wherein the surgical device is a probe insertable through a cannula.
5. The system according to 4, wherein the probe is selected from the group consisting of an electrode, a microwave antenna, an optical fiber, and a cryoablation probe.
6. The system according to 4 or 5, wherein the probe includes proximal and distal ends, the distal end of the probe selectively advanceable to expose the distal end of the probe from the distal end of the cannula.
7. The system according to any preceding item, wherein the library of thermal images includes thermal images selected from a group consisting of an actual thermal profile of the surgical device and a thermal profile derived from computer simulating techniques.
8. The system according to any preceding item, wherein the querying algorithm is configured to locate, orient and scale the thermal images according to the surgical device in situ.
9. The system according to any preceding item, wherein the graphical user interface module comprises an electronic pointing device.
10. The system according to 9, wherein the electronic pointing device is used to identify and outline the thermal image of the surgical device in situ.
11. The system according to any preceding item, wherein the imaging device is operatively associated with the library of thermal images to allow the selectable imposition of a selected simulated thermal image within a target surgical site.
12. The system according to any preceding item, wherein the querying algorithm is configured to select computer generated predicted images according to a desired treatment plan for the surgical device based on at least one of an electrical setting of the generator, a tip configuration of the surgical device, a depth of penetration of the surgical device, an activation time of the surgical device, and combinations thereof.
13. A method for thermally treating target tissue, the method comprising the steps of:
   selecting a surgical device adapted to connect to a surgical generator;
   displaying an image of the surgical device in situ;
   connecting to a database module adapted to connect to a library of thermal images;
   querying simulated thermal images according to a desired thermal image profile for the surgical device based on at least one of an electrical setting of the generator, a tip configuration of the surgical device, a depth of penetration of the surgical device, an activation time of the surgical device, and combinations thereof; and
   superimposing a selected simulated thermal image atop the image of the surgical device and displaying both the image of the surgical device in situ and the simulated thermal image on an imaging device over the surgical device in situ.
14. The method according to 13, further comprising the step of identifying and outlining a simulated image on the imaging device.
15. The method according to 13 or 14, further comprising the step of querying computer generated predicted overlays according to at least one of the size, shape and type of procedure and surgical device selected.
16. The method according to 13, 14, or 15, further comprising the step of displaying an option for a user to adjust the size of tissue treatment volume.
17. The method according to 13, 14, 15, or 16, further comprising the step of monitoring and controlling the supply of electrosurgical energy of the generator via a thermal sensor feedback loop.
18. The method of 13, 14, 15, 16, or 17, wherein the database module may contain at least one of an overlay, a computer generated simulation, and a video of previously performed surgical procedures.

## Claims

1. A system (500) for thermally treating target tissue, the system comprising:
a surgical device (104) adapted to connect to a surgical generator;
an imaging device (304) operable to display an image of the surgical device in situ (104');
a database module (225) adapted to connect to a library (200) of thermal images (202, 508);
a querying algorithm (502) configured to select a simulated thermal image (510') according to a desired thermal image for the surgical device based on at least one of an electrical setting of the generator, a tip configuration of the surgical device, a depth of penetration of the surgical device, an activation time of the surgical device, and combinations thereof; and
a graphical user interface module configured to overlay the selected simulated thermal image over the image of the surgical device in situ;
**characterized in that**
the querying algorithm is configured such that if no appropriate thermal image is available in the library, one or more thermal images are extrapolated or scaled to achieve an approximate desired thermal image.

2. The system according to claim 1, wherein the graphical user interface module comprises an electronic pointing device (518).

3. The system according to claim 2, wherein the electronic pointing device (518) is configured to be used to identify and outline the thermal image of the surgical device in situ.

4. A system in accordance with any preceding claim, wherein the querying algorithm is configured to query computer generated predicted overlays (508) according to at least one of the size, shape, and type of surgical device.

5. A system in accordance with any preceding claim, wherein the querying algorithm is configured to locate, orient and scale the thermal image to correspond with the surgical device in situ.

6. A system in accordance with any preceding claim, wherein the graphical use interface module is configured to display an option (602, 604) for a user to adjust the size of tissue treatment volume.

7. A system in accordance with any preceding claim, wherein the database module contains at least one of an overlay, a computer generated simulation, and a video of previously performed surgical procedures.

8. The system according to any preceding claim, further comprising a thermal sensor feedback loop for monitoring and controlling the supply of electrosurgical energy of the generator.

## Patentansprüche

1. System (500) zum thermischen Behandeln von Zielgewebe, das System aufweisend:
eine chirurgische Vorrichtung (104), die zum Verbinden mit einem chirurgischen Generator geeignet ist;
eine Bildgebungsvorrichtung (304), die zum Anzeigen eines Bildes der chirurgischen Vorrichtung in situ (104') betriebsbereit ist;
ein Datenbankmodul (225), das zum Verbinden mit einer Bibliothek (200) von thermischen Bildern (202, 508) geeignet ist;
ein Abfragealgorithmus (502), der konfiguriert ist zum Auswählen eines simulierten thermischen Bildes (510') gemäß einem gewünschten thermischen Bild für die chirurgische Vorrichtung basierend auf wenigstens einem von einer elektrischen Einstellung des Generators, einer Spitzenkonfiguration der chirurgischen Vorrichtung, einer Eindringtiefe der chirurgischen Vorrichtung, einer Aktivierungszeit der chirurgischen Vorrichtung und Kombinationen davon; und
ein grafisches Benutzeroberflächenmodul, die konfiguriert ist zum Überlagern des ausgewählten simulierten thermischen Bildes mit dem Bild der chirurgischen Vorrichtung in situ;
**dadurch gekennzeichnet, dass**
der Abfragealgorithmus konfiguriert ist, so dass, wenn kein geeignetes thermisches Bild in der Bibliothek verfügbar ist, eines oder mehrere thermische Bilder hochgerechnet oder skaliert werden, um ein angenähertes gewünschtes thermisches Bild zu erhalten.

2. System gemäß Anspruch 1, wobei das grafische Benutzeroberflächenmodul eine elektronische Zeigervorrichtung (518) aufweist.

3. System gemäß Anspruch 2, wobei die elektronische Zeigervorrichtung (518) konfiguriert ist um dazu verwendet zu werden das thermische Bild der chirurgischen Vorrichtung in situ zu identifizieren und zu skizzieren.

4. System in Übereinstimmung mit einem vorangegangenen Anspruch, wobei der Abfragealgorithmus konfiguriert ist zum Abfragen von computergenerierten Überlagerungen (508) gemäß wenigstens einem von der Größe, der Form und dem Typ der chirurgischen Vorrichtung.

5. System in Übereinstimmung mit einem vorangegangenen Anspruch, wobei der Abfragealgorithmus konfiguriert ist, um das thermische Bild zu platzieren, auszurichten und zu skalieren zum Korrespondieren mit der chirurgischen Vorrichtung in situ.

6. System in Übereinstimmung mit einem vorangegangenen Anspruch, wobei das grafische Benutzeroberflächenmodul konfiguriert ist, um für einen Benutzer eine Option (602, 604) zum Anpassen der Größe eines Gewebebehandlungsvolumens anzuzeigen.

7. System in Übereinstimmung mit einem vorangegangenen Anspruch, wobei das Datenbankmodul wenigstens eines von einer Überlagerung, einer computergenerierten Simulation und einem Video von zuvor durchgeführten chirurgischen Eingriffen enthält.

8. System gemäß einem vorangegangenen Anspruch, weiter aufweisend eine thermische Sensorfeedbackschleife zum Überwachen und Steuern der elektrochirurgischen Energieversorgung des Generators.

## Revendications

1. Système (500) pour traiter thermiquement un tissu cible, le système comprenant :
un dispositif chirurgical (104) apte à être connecté à un générateur chirurgical ;
un dispositif d'imagerie (304) pouvant être mis en oeuvre pour afficher une image du dispositif chirurgical in situ (104') ;
un module de base de données (225) apte à être connecté à une bibliothèque (200) d'images thermiques (202, 508) ;
un algorithme d'interrogation (502) configuré pour sélectionner une image thermique simulée (510') selon une image thermique souhaitée pour le dispositif chirurgical sur la base d'au moins un parmi un réglage électrique du générateur, une configuration de pointe du dispositif chirurgical, une profondeur de pénétration du dispositif chirurgical, un temps d'activation du dispositif chirurgical, et des combinaisons de ceux-ci ; et
un module d'interface utilisateur graphique configuré pour superposer l'image thermique simulée sélectionnée sur l'image du dispositif chirurgical in situ ;
**caractérisé en ce que** :
l'algorithme d'interrogation est configuré de sorte que si aucune image thermique appropriée n'est disponible dans la bibliothèque, une ou plusieurs images thermiques sont extrapolées ou mises à l'échelle pour parvenir à une image thermique souhaitée approchée.

2. Système selon la revendication 1, dans lequel le module d'interface utilisateur graphique comprend un dispositif de pointage électronique (518).

3. Système selon la revendication 2, dans lequel le dispositif de pointage électronique (518) est configuré pour être utilisé pour identifier et indiquer l'image thermique du dispositif chirurgical in situ.

4. Système selon l'une quelconque des revendications précédentes, dans lequel l'algorithme d'interrogation est configuré pour interroger des incrustations prédites produites par ordinateur (508) selon au moins un parmi la taille, la forme et le type de dispositif chirurgical.

5. Système selon l'une quelconque des revendications précédentes, dans lequel l'algorithme d'interrogation est configuré pour localiser, orienter et mettre à l'échelle les images thermiques pour correspondre au dispositif chirurgical in situ.

6. Système selon l'une quelconque des revendications précédentes, dans lequel le module d'interface utilisateur graphique est configuré pour afficher une option (602, 604) pour qu'un utilisateur ajuste la taille du volume de traitement de tissu.

7. Système selon l'une quelconque des revendications précédentes, dans lequel le module de base de données contient au moins un d'une incrustation, d'une simulation produite par ordinateur, et d'une vidéo de procédures chirurgicales précédemment effectuées.

8. Système selon l'une quelconque des revendications précédentes, comprenant en outre une boucle de rétroaction de capteur thermique pour contrôler et commander la fourniture d'énergie électrochirurgicale du générateur.
